Europäisches Patentamt

European Patent Office

Office européen des brevets

(19)

(11) **EP 0 897 312 B1**

(12) **FASCICULE DE BREVET EUROPEEN**

(45) Date de publication et mention
de la délivrance du brevet:
**05.07.2000 Bulletin 2000/27**

(21) Numéro de dépôt: **97918229.2**

(22) Date de dépôt: **15.04.1997**

(51) Int Cl.⁷: $A61N\ 1/30$

(86) Numéro de dépôt international:
**PCT/FR97/00672**

(87) Numéro de publication internationale:
**WO 97/38750 (23.10.1997 Gazette 1997/45)**

(54) **DISPOSITIF D'ADMINISTRATION TRANSDERMIQUE DE MEDICAMENTS PAR IONOPHORESE**

VORRICHTUNG ZUR TRANSDERMALEN ABGABE VON MEDIKAMENTEN DURCH
IONTOPHORESE

DEVICE FOR TRANSCUTANEOUS ADMINISTRATION OF MEDICATIONS USING
IONOPHORESIS

(84) Etats contractants désignés:
**AT BE CH DE DK ES FI FR GB GR IE IT LI LU MC
NL PT SE**

(30) Priorité: **16.04.1996 FR 9604735**

(43) Date de publication de la demande:
**24.02.1999 Bulletin 1999/08**

(73) Titulaire: **LABORATOIRES D'HYGIENE ET
DE DIETETIQUE L.H.D.
75008 Paris (FR)**

(72) Inventeur: **MILLOT, Philippe
F-21000 Dijon (FR)**

(74) Mandataire: **Colas, Jean-Pierre
Cabinet de Boisse et Colas
37, avenue Franklin D. Roosevelt
75008 Paris (FR)**

(56) Documents cités:
**EP-A- 0 461 680          WO-A-96/10440**

**Description**

La présente invention est relative à un dispositif d'administration transdermique de médicaments par ionophorèse, et, plus particulièrement, à un tel dispositif comprenant au moins a) un ensemble incluant une électrode accolée à un réservoir chargeable avec un médicament, et une contre-électrode, et, b) un module électronique montable, de manière séparable, sur ledit ensemble pour commander dans le temps l'intensité d'un courant électrique traversant, entre les deux électrodes, le réservoir et la peau d'un patient appliquée contre ledit réservoir.

On connaît de tels dispositifs, notamment des demandes internationales de brevet WO 94/27671, WO 94/28965 et de la demande de brevet français n° 2 656 223. Ils sont conçus, notamment, pour l'administration de médicaments appartenant à trois classes thérapeutiques : les antalgiques majeurs, les antiémétiques et les antimigraineux. La réponse des patients aux antalgiques majeurs, tels que le fentanyl et ses dérivés par exemple, est très variable. Il s'ensuit que le traitement doit être adapté au patient par le médecin. Les dispositifs ionophorétiques d'administration de médicaments conviennent particulièrement bien dans cette application, du fait que les programmes d'administration qu'ils permettent d'exécuter peuvent être extrêmement variés, tout en étant susceptibles d'être modifiés avec une très grande souplesse.

Encore faut-il éviter tout risque d'erreurs dans le traitement, notamment dans le cas d'antalgiques extrêmement actifs tels que le fentanyl et ses dérivés, qui peuvent être dangereux, voire fatals lorsqu'ils sont administrés à des doses qui s'écartent de celles précisément déterminées par les études pharmacologiques. Pour cela il faut faire en sorte d'être assuré que le médicament contenu dans le réservoir est bien celui qui a été choisi et que l'administration de ce médicament s'exécutera suivant un programme contrôlé et lancé par une personne compétente. Il faut donc que l'accès aux moyens de configuration et de modification de ce programme soit réservé à une telle personne pour empêcher que des interventions maladroites ne créent un danger pour le patient.

Dans cet ordre d'idées, la demande de brevet français n° 2 656 223 précitée propose que le module électronique soit chargé avec tous les programmes d'administration de médicaments correspondant aux divers ensembles d'électrodes qu'il est susceptible de recevoir, la sélection d'un programme particulier s'opérant, au moment du montage d'un ensemble d'électrodes particulier sur le module, à l'aide de moyens de contact mécaniques ou électriques disposés en interface entre l'ensemble et le module, pour sélectionner automatiquement un programme applicable au médicament contenu dans le réservoir de l'ensemble d'électrodes. En variante, la demande de brevet français précitée propose que la sélection se fasse par lecture, par le module électronique, d'un code à barres porté par l'ensemble d'électrodes, lors de l'assemblage de ces deux éléments. Aucun moyen n'est prévu pour empêcher l'emploi de ce dispositif par une personne non autorisée.

En outre, l'utilisation d'un code à barres limite la quantité d'informations qui peut être transférée de l'ensemble d'électrodes vers le module électronique. Elle oblige à prévoir, dans le module, des moyens de lecture du code à barres, cette lecture pouvant être empêchée par interposition de matériaux opaques au faisceau de lecture. Un code à barres peut en outre être facilement reproduit par photocopie, ce qui rend possibles des falsifications éventuellement dangereuses.

La sélection d'un programme par des moyens mécaniques tels que des saillies de commande d'interrupteurs électriques distribués suivant une configuration particulière, ou par des moyens purement électriques, présente l'inconvénient de mettre en jeu des configurations figées de saillies ou de contacts électriques, qui peuvent être facilement identifiées, copiées ou activées par des personnes non autorisées.

On connaît de WO 96/10440 un dispositif ionophorétique d'administration de médicaments comprenant un module électronique de commande, un ensemble d'électrodes et des moyens électroniques de lecture de codes pour vérifier la compatibilité d'un ensemble d'électrodes particulier avec ledit module.

La présente invention a pour but de réaliser un dispositif ionophorétique d'administration de médicaments conçu de manière à assurer la sécurité de l'administration, tant en ce qui concerne la compétence de l'administrateur que la sélection du produit administré et du programme d'administration.

La présente invention a aussi pour but de réaliser un tel dispositif qui soit cependant très versatile dans sa programmation et sa reprogrammation éventuelle.

On atteint ces buts de l'invention, ainsi que d'autres qui apparaîtront à la lecture de la description qui va suivre, avec un dispositif d'administration transdermique de médicaments par ionophorèse, comprenant au moins premièrement un ensemble incluant une électrode accolée à un réservoir chargeable avec un médicament, et une contre-électrode, deuxièmement un module électronique montable, de manière séparable, sur ledit ensemble pour commander dans le temps l'intensité d'un courant électrique thérapeutique traversant, entre les deux électrodes, le réservoir et la peau d'un patient appliquée contre ledit réservoir, ledit module électronique comprenant des moyens de commande du fonctionnement du dispositif, celui-ci étant remarquable en ce qu'il comprend troisièmement une clé électronique incluant une mémoire chargée avec un code prédéterminé et des moyens de connexion temporaire au module électronique pour la lecture de ce code par des moyens de lecture présents dans ce module, les moyens de commande dudit module étant sensibles au code de ladite clé pour autoriser sélectivement le fonctionnement du dispositif et en ce que la clé forme un élément distinct des deux autres.

Selon un mode de réalisation préféré du dispositif suivant l'invention, l'ensemble d'électrodes comprend une mémoire électronique chargée avec un code prédéterminé. Les moyens de lecture comprennent des moyens de lecture dudit code et lesdits moyens de commande prennent aussi en compte le code dudit ensemble pour autoriser sélectivement le fonctionnement du dispositif.

Comme on le verra plus loin, la présence d'une mémoire dans l'ensemble d'électrodes permet de conserver dans celle-ci un code identifiant le médicament contenu dans le réservoir. La sûreté de la lecture de ce code par le module est renforcée et son décryptage par des personnes non autorisées est rendu plus difficile que dans la technique antérieure exposée ci-dessus.

Une telle mémoire est susceptible de conserver un code d'identification choisi parmi un nombre presque illimité de tels codes, ce qui est avantageux dans le cadre d'une fabrication industrielle. En outre, comme on le verra plus loin, cette mémoire est susceptible de recevoir des informations autres qu'un tel code, un programme d'administration transférable dans le module électronique par exemple, ce qui donne au dispositif suivant l'invention une grande versatilité.

Ainsi l'administration de médicaments n'est rendue possible que si le programme d'administration sélectionné est celui qui correspond au médicament contenu dans l'ensemble d'électrodes, et si cette administration est voulue par une personne habilitée, médecin compétent ou patient autorisé. Comme la mémoire de l'ensemble d'électrodes, celle de la clé accroît en outre la versatilité du dispositif en accueillant éventuellement, elle aussi, des programmes d'administration.

D'autres caractéristiques et avantages de la présente invention apparaîtront à la lecture de la description qui va suivre et à l'examen du dessin annexé dans lequel :

- la figure 1 représente en A un ensemble d'électrodes, en B un module électronique et en C une clé électronique qui constituent les éléments du dispositif suivant l'invention,
- la figure 2 est un schéma fonctionnel illustrant la structure et le fonctionnement de ces trois éléments, et
- les figures 3 et 4 sont des organigrammes de programmes d'instructions exécutés par le module électronique du dispositif suivant l'invention.

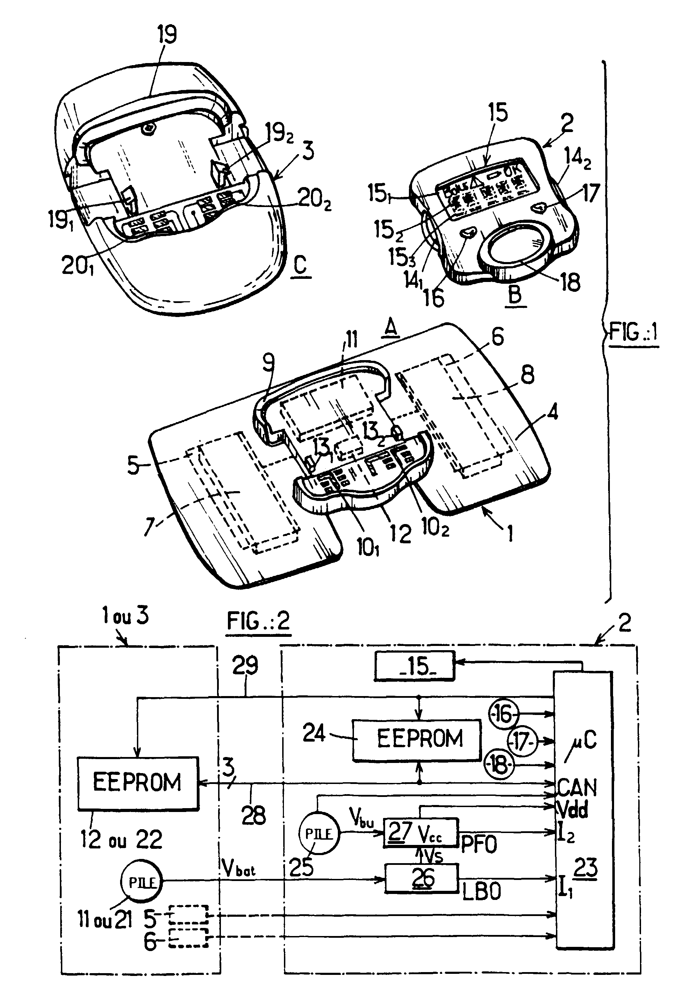

Celui-ci comprend essentiellement trois éléments distincts : un ensemble d'électrodes 1, un module électronique 2 et une "clé" électronique, représentés respectivement en A, B, C à la figure 1.

L'ensemble d'électrodes 1 comprend une feuille souple 4, en matière plastique par exemple, garnie sur sa face cachée du point de vue de la figure 1, A, de deux électrodes métalliques 5 et 6 accolées à des réservoirs 7, 8 respectivement, constitués par exemple par une couche d'un hydrogel. L'un au moins des réservoirs est conçu pour recevoir, en solution, des molécules ionisées d'un principe actif.

Pendant une administration de ce principe actif à un patient, les deux électrodes sont plaquées sur sa peau par des moyens tels qu'un bracelet. Le module électronique 2 est alors reçu dans un berceau 9 conforme, fixé sur l'autre face de la feuille 4. Le module commande alors l'établissement d'une tension électrique entre les électrodes 5 et 6, de manière que les molécules de principe actif soient forcées sous la peau du patient par le champ électrique établi entre les deux électrodes, comme cela est bien connu dans la technique de l'administration transdermique de médicaments par ionophorèse.

Les liaisons électriques nécessaires entre le module électrique 2 et l'ensemble d'électrodes 1 sont établies par des jeux de contacts électriques complémentaires présents sur ces deux éléments, contacts qui se ferment lors de la mise en place du module sur le berceau 9 de l'ensemble d'électrodes 1, les contacts portés par ce dernier étant visibles en $10_1$, $10_2$ dans ce berceau. Le fond du berceau 9 reçoit en outre, du côté qui fait face à la feuille 4, une pile électrique 11 et une puce 12 de mémoire électronique, dont on expliquera le rôle plus loin.

Le berceau 9 comprend encore des dents d'encliquetage $13_1$, $13_2$ qui coopèrent avec des touches flexibles $14_1$, $14_2$ formées sur le module 2 (voir figure 1, B) pour solidariser mécaniquement le module à son berceau de réception sur l'ensemble 1, une pression sur ces touches $14_1$, $14_2$ permettant de désolidariser le module de ce berceau.

Sur la vue extérieure de la figure 1, B, il apparaît que le module 2 comprend en outre un écran d'affichage 15, à cristaux liquides par exemple, et des boutons poussoirs 16, 17, 18.

L'écran 15 permet d'afficher, sur trois rangées superposées $15_1$, $15_2$, $15_3$, des icônes et des messages.

Une pression sur le bouton-poussoir 16 fait apparaître un message "INFO" sur la ligne $15_3$. Ce bouton est actionné pour obtenir l'affichage d'informations contenues dans le module 2, telles que l'heure, la dose de médicament à administrer, en milligrammes/heure par exemple. Ces informations sont affichées sur la ligne $15_2$, qui comporte une rangée de plages d'affichage de caractères alphanumériques. Cette ligne, à cinq caractères alphanumériques par exemple, permet d'afficher aussi d'autres messages tels qu'un message d'initialisation de processus, un code d'erreurs, etc, etc...

Une pression sur le bouton-poussoir 17 fait apparaître un message "PROGRAMME" sur la ligne $15_3$. Le médecin actionne ce bouton pour entrer ou modifier des programmes d'administration de médicaments, dans la mesure où il y est autorisé par les procédures que l'on décrira plus loin.

Le bouton-poussoir 18 peut être pressé par un patient en cours de traitement. Il permet de commander l'administration d'une dose supplémentaire ou "bolus" de médicament, par exemple lorsque le patient souhaite atténuer rapidement une vive douleur survenant inopinément. Lorsqu'un tel bolus est demandé, le message "BOLUS" apparaît sur la ligne $15_1$ de l'écran 15. Cette ligne peut afficher aussi des icônes pour alerter le patient ou le médecin sur la survenance d'une situation dangereuse, ou sur une défaillance d'une pile d'alimentation électrique du module, de l'ensemble d'électrodes, ou de la clé. Elle peut aussi afficher un message "OK" pour signaler qu'un traitement peut commencer, ou d'autres messages ou icônes utiles.

Comme on l'a vu plus haut, la présente invention a pour but de sécuriser au mieux l'administration transdermique d'un médicament par ionophorèse, vis-à-vis d'erreurs éventuelles dans la nature du médicament administré ou dans le programme d'administration de ce médicament, notamment quand ces erreurs peuvent être dangereuses ou fatales pour le patient. A cet effet, le dispositif suivant l'invention n'autorise l'administration d'un traitement qu'après exécution d'une procédure de reconnaissance mutuelle, au moins entre l'ensemble d'électrodes et le module électronique, procédure que l'on décrira dans la suite de la présente description.

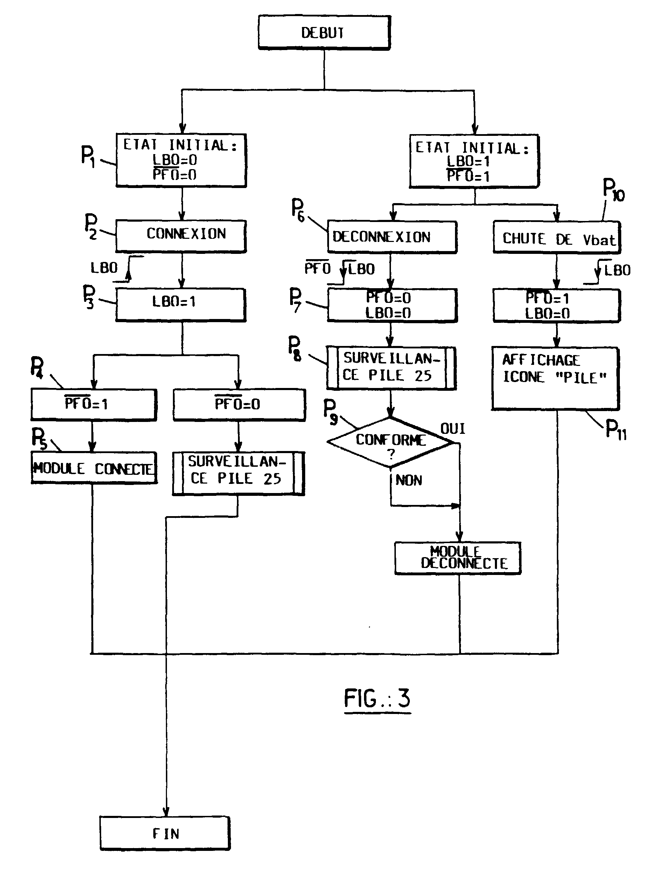

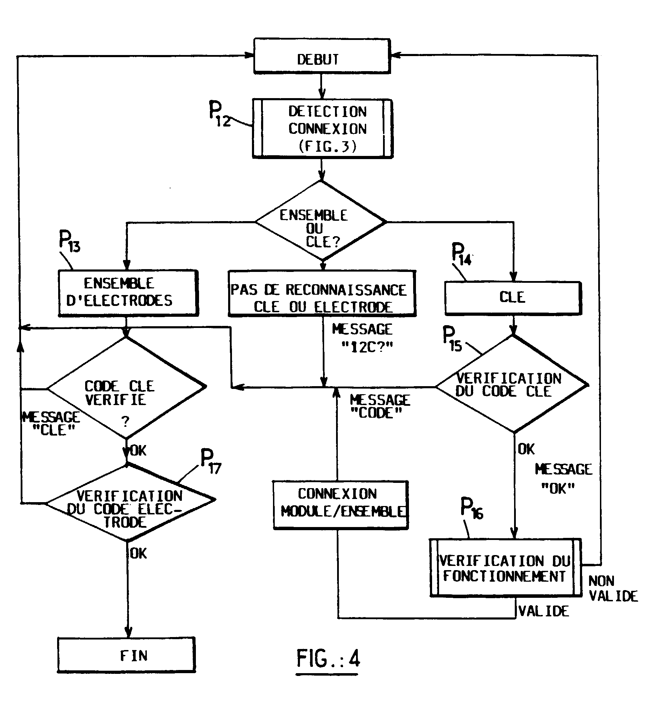

Pour sécuriser encore plus l'administration du médicament, lorsque cela est souhaitable ou nécessaire, la présente invention permet de vérifier en outre que le traitement est décidé par une personne compétente et autorisée par la détention de la clé électronique 3. Dans ce cas, le dispositif suivant l'invention exécute une procédure de reconnaissance mutuelle de ces trois éléments, préalablement à l'administration du médicament. Cette administration ne peut alors démarrer que si elle est autorisée par cette procédure, décrite ci-après en liaison avec les organigrammes des figures 3 et 4.

L'exécution de cette procédure exige que le module électronique soit couplé, successivement, à la clé 3 et à l'ensemble d'électrodes 1. A cet effet, la clé 3 comprend un berceau 19 de fond conforme à celui du module 2, ce berceau 19 étant muni, comme le berceau 9 de l'ensemble 1, de deux jeux de contacts électriques $20_1$, $20_2$ pour assurer le couplage électrique de la clé et du module.

La clé 3 comprend encore, comme l'ensemble 1, une pile d'alimentation électrique 21 et une mémoire électronique 22 qui n'apparaissent pas sur la figure 1,C mais qui sont schématisées à la figure 2, de même que tous les autres moyens électriques et électroniques du dispositif suivant l'invention , nécessaires à l'exécution de la procédure de reconnaissance mutuelle des trois éléments du dispositif.

Sur cette figure 2, on a schématisé la connexion du module électronique 2 à l'un ou l'autre de l'ensemble d'électrodes 1 et de la clé 3. C'est ainsi que le bloc schématisant l'un de ces éléments 1 et 3 comprend une pile d'alimentation 11 ou 21 et une mémoire 12 ou 22, suivant qu'il s'agit de l'ensemble 1 ou de la clé 3.

Le module électronique 2 comprend essentiellement un régisseur de processus 23 couramment appelé "microcontrôleur", une mémoire électronique 24, une pile 25 d'alimentation électrique de sauvegarde du module, délivrant une tension de "back-up" $V_{bu}$ et des moyens de commande (26, 27) de l'alimentation électrique du microcontrôleur 23. Incidemment, le microcontrôleur 23 commande aussi, directement ou indirectement, l'afficheur 15.

Par ailleurs, un bus de communication 28 permet au microcontrôleur 23 de lire et/ou écrire dans les diverses mémoires 12, 22, 24 du dispositif suivant l'invention. Ces mémoires peuvent être, à titre d'exemple illustratif et non limitatif, du type EEPROM, les communications s'effectuant à l'aide du bus 28 qui peut être, par exemple, un bus maître-esclave bidirectionnel synchrone à trois fils du type I2C. L'activation des diverses mémoires s'opère alors à l'aide d'une ligne d'activation 29 commandée par le microcontrôleur 23.

Suivant l'invention, les moyens de commande (26, 27) sont connectés à la fois à la pile 25 de sauvegarde de l'alimentation du microcontrôleur 23 et à la pile 11 ou 21 placée dans l'ensemble 1 ou la clé 3, quand l'un ou l'autre de ces éléments est couplé au module 2. En l'absence d'un tel couplage, l'alimentation du microcontrôleur, et notamment de sa mémoire vive interne, est assurée par la pile 25 pour éviter l'effacement de données contenues dans cette mémoire. Par contre, suivant l'invention, quand il y a couplage, le module est avantageusement alimenté par la pile 11 ou 21 de l'élément auquel il est couplé, de manière à ménager alors sa pile 25 de sauvegarde, au moins quand la tension délivrée par la pile 11 ou 21 est suffisante pour assurer la fourniture d'une tension d'alimentation $V_{dd}$ convenable au microcontrôleur. C'est le rôle des moyens (26, 27) que de sélectionner et de contrôler les piles qui assurent cette alimentation.

Pour ce faire, suivant un mode de réalisation de l'invention donné seulement à titre d'exemple, ces moyens (26,27) de commande d'alimentation sont constitués par deux circuits intégrés du commerce, respectivement un régulateur linéaire 26 et un circuit superviseur 27 référencés respectivement MAX 884 et MAX 690 dans les catalogues de la société MAXIM INTEGRATED PRODUCTS de Sunnyvale, CA, USA.

Le régulateur 26 comprend une broche de sortie numérique LBO qui est à l'état haut quand le régulateur est alimenté, c'est-à-dire quand il reçoit une tension suffisante de la pile 11 ou 21 de l'élément 1 ou 3 du dispositif respectivement, qui est couplé au module 2. Le signal LBO est délivré à une entrée d'interruption $I_1$ du microcontrôleur 23 pour informer ce dernier, soit d'une connexion correcte à la pile 11 ou 21 délivrant une tension $V_{bat}$ supérieure à la tension d'alimentation minimale (4,5 volts par exemple) du régulateur 26 (LBO = 1) soit une déconnexion ou une tension d'alimentation $V_{bat}$ insuffisante ($V_{bat}$ < 4,5 volts), situations dans lesquelles LBO = 0. Quand LBO = 1, le régulateur 26 alimente le superviseur 27 avec une tension stabilisée $V_s$ de 3,3 volts. Pour ce faire, un pont diviseur (non représenté) extérieur au régulateur 26 est alimenté par la tension + $V_{bat}$ délivrée par la pile. Le rapport de division du pont est choisi pour qu'une tension + $V_{bat}$ = 4,5 volts produise une tension de sortie du pont égale à 1,2 volt. Cette tension alimente un comparateur interne au régulateur, dont l'autre broche est alimentée par une tension de référence interne de 1,2 volt. La sortie de ce comparateur constitue le signal LBO.

Le superviseur 27 est alimenté en outre par la pile 25 qui lui fournit une tension nominale $V_{bu}$ de 3 volts. Le rôle du superviseur est de sélectionner, pour l'alimentation du microcontrôleur 23 qui doit recevoir une tension $V_{dd}$ au moins égale à 2,4 volts, soit la tension $V_s$ délivrée par le régulateur si celle-ci est présente et suffisante, soit la tension $V_{bu}$ dans le cas contraire. Le superviseur 27 a une broche de sortie $\overline{PFO}$ qui se trouve à l'état haut quand la tension $V_s$ délivrée par le régulateur est supérieure à 2,4 volts et que $V_{bu}$ est supérieure à 2 volts. La sortie $\overline{PFO}$ est connectée à une autre entrée d'interruption $I_2$ du microcontrôleur 23. Quand $V_{bu}$ < 2 V alors que 2,4 V < $V_{bat}$ < 4,5 V, $\overline{PFO}$ et LBO sont tous deux à l'état bas. Le microcontrôleur 23 sait par le bus 28 s'il y a connexion ou non du module 2 à l'ensemble d'électrodes 1 ou à la clé 3 mais ne peut déterminer si l'état $\overline{PFO}$ = LBO = 0 est dû à $V_{bu}$ < 2 volts ou à 2,4 volts < $V_{bat}$ < 4,5 volts. Pour lever cette indétermination, le microcontrôleur 23 est programmé pour réagir à $\overline{PFO}$ = LBO = 0 par une mesure directe de la tension $V_{bu}$ délivrée par la pile, à travers l'entrée CAN d'un convertisseur analogique-numérique intégré au microprocesseur. Si la mesure indique $V_{bu}$ < 2 volts, le microcontrôleur 23 commande l'affichage d'un message sur l'écran 15 pour signaler que la pile 25 est à changer. Si $V_{bu}$ est supérieur à 2 volts, cela signifie que la tension $V_{bat}$ délivrée par la pile 11 ou 21 est telle que 2,4 V < $V_{bat}$ < 4,5 V. Le microcontrôleur commande alors l'affichage d'un message invitant l'utilisateur à remplacer cette pile.

Ainsi, les moyens (26, 27) permettent au microcontrôleur 23 de savoir à tout instant si le module 2 qui le contient est ou non connecté électriquement et mécaniquement à un élément extérieur, clé 3 ou ensemble d'électrodes 1, sans pour autant déterminer lequel. Cette capacité est utilisée par le dispositif suivant l'invention dans le processus de reconnaissance mutuelle de ses trois éléments : module, ensemble d'électrodes, clé, tel que ce processus est illustré par les organigrammes des figures 3 et 4 que l'on décrira plus loin en détail.

Il faut compléter préalablement la description précédente du dispositif suivant l'invention, faite en liaison avec la figure 2. A cet égard, il faut rappeler que, lorsque le module 2 est monté sur l'ensemble 1, il existe une liaison électrique entre le module 1 et les électrodes 5 et 6 de l'ensemble 1. En effet, le module 2 commandant l'intensité du courant électrique passant entre ces deux électrodes 5 et 6, une mesure de la tension entre électrodes est nécessaire au module pour réguler cette intensité à la valeur qu'il a déterminée.

On va maintenant décrire la procédure de reconnaissance mutuelle des divers éléments du dispositif suivant l'invention, en liaison avec les organigrammes des programmes illustrés aux figures 3 et 4, exécutés par le microcontrôleur 23, dûment programmé à cet effet.

Ce processus s'exécute alors qu'un patient, ou un praticien, autorisé, détenteur de la clé, et désireux de déclencher un traitement, connecte successivement le module électronique 2 à cette clé 3 puis à l'ensemble d'électrodes 1 pour lancer le programme d'administration du médicament, si cette administration est autorisée par la procédure. La procédure se déclenche automatiquement lors des connexions clé/module et module/ensemble d'électrodes. La procédure doit donc incorporer une phase de reconnaissance des connexions, illustrée par l'organigramme de la figure 3, qui met en jeu essentiellement les signaux LBO et $\overline{PFO}$ définis ci-dessus et qui présentent les états suivants :

LBO = 1 si $V_{bat}$ > 4,5 volts

LBO = 0 si $V_{bat}$ < 4,5 volts

$\overline{PFO}$ = 0 si $V_{bat}$ < 2,4 volts
      ou si $V_{bu}$ < 2 volts

$\overline{PFO}$ = 1 si $V_{bat}$ > 2,4 volts
      et $V_{bu}$ > 2 volts

Avant une connexion clé/module ou module/ensemble d'électrodes, on a LBO = $\overline{PFO}$ = 0 (pas $P_1$ de l'organigramme). Au pas $P_2$, le patient ou le médecin établit une connexion qui peut être aussi bien une connexion clé/module qu'une connexion module/ensemble d'électrodes. Cette connexion fait passer LBO de 0 à 1 (pas $P_3$) si la tension Vbat délivrée par la pile 11 ou 21 de l'ensemble 1 ou de la clé 3 est supérieure à 4,5 volts. Si Vbat < 4,5 volts, LBO reste à zéro et un message signalant la faiblesse de la pile s'affiche sur l'écran 15, comme indiqué plus haut. La séquence redémarre avant le pas $P_1$. Au pas $P_4$, le microcontrôleur teste $\overline{PFO}$. Si $\overline{PFO}$ est passé à 1, c'est que le régulateur 26 délivre bien $V_s$ = 3,3 volts et que $V_{bu}$ est supérieur à 2 volts. Le microprocesseur en déduit (pas $P_5$) que le module est connecté à l'ensemble 1 ou à la clé 3, sans pouvoir préciser s'il s'agit de l'un ou de l'autre. Dans le cas contraire, ($\overline{PFO}$ = 0), cela signifie que $V_{bu}$ est plus petit que 2 volts et qu'il y a lieu de changer la pile 25 du module, épuisée.

A la séparation du module et de l'élément (clé ou ensemble d'électrodes) du dispositif qui lui est connecté, on peut encore tirer des conclusions des évolutions des signaux LBO et $\overline{PFO}$, quant à l'intervention de cette déconnexion et quant à l'état des piles du dispositif. Partant d'un état initial ou LBO = $\overline{PFO}$ = 1, significatif d'un état de connexion avec des tensions $V_{bat}$ et $V_{bu}$ adéquates, nécessaires au lancement et à l'exécution d'un traitement, cet état initial peut être modifié, soit par déconnexion à la fin du traitement par exemple (pas $P_6$), soit par une chute de la tension $V_{bat}$ fournie pendant le traitement au module 2 par l'ensemble 1 ou la clé 3 (pas $P_7$), en dessous du seuil de 4,5 volts. Dans le cas d'une déconnexion, on lit, au pas $P_7$, $\overline{PFO}$ = LBO = 0. Le passage de $\overline{PFO}$ à l'état bas déclenche (pas $P_8$) un test de la tension $V_{bu}$ fournie par la pile 25, contrôlée à travers le convertisseur analogique-numérique intégré au microcontrôleur 23. Ainsi, outre l'information de déconnexion reçue par le microcontrôleur lors du passage de LBO à l'état bas, ce test renseigne sur la nécessité ou non de changer la pile 25 de sauvegarde (pas $P_9$). Le microcontrôleur affiche alors une icône sur l'écran 25 pour signaler cette situation au patient et/ou au médecin.

Au cours d'un traitement démarré alors que LBO = $\overline{PFO}$ = 1, il peut arriver que la pile 11 de l'ensemble d'électrodes, qui fournit le courant électrique nécessaire au traitement sous la commande du microcontrôleur, s'épuise (pas $P_{10}$). On observe alors que LBO passe à l'état bas. Au pas $P_{11}$, le microcontrôleur commande l'affichage d'une icône sur l'écran 15 du module pour alerter le patient ou le médecin sur la nécessité de changer cette pile 11, comme on l'a vu plus haut.

Le processus décrit ci-dessus permet au microcontrôleur 23 du module 2 d'être renseigné à tout instant sur son état de connexion/déconnexion à un autre élément du dispositif et sur l'état des piles du module et de cet élément.

Ayant détecté une connexion, le microcontrôleur 23 du module 2 peut alors lancer une procédure de reconnaissance de l'élément du dispositif (1, 2, 3) qui est connecté au module, et de vérification de la compatibilité de cet élément avec le module. Pour ce faire, le microcontrôleur doit lire et comparer des informations contenues dans les trois mémoires EEPROM 12, 22, 24 du dispositif. Chaque mémoire possède ainsi une adresse particulière et stocke au moins un code sur lequel va porter le processus de reconnaissance de compatibilité des éléments du dispositif. Elle peut aussi recevoir un ou plusieurs programmes d'administration de médicament, exécutables sous la commande du microcontrôleur. C'est le cas notamment des mémoires 12 et 24 de l'ensemble d'électrodes 1 et du module 2. La mémoire 22 de la clé peut aussi recevoir un tel programme, comme on le verra plus loin.

Un adressage sur 2 bits $E_1E_2$ suffit pour permettre au microcontrôleur 23 d'accéder sélectivement à ces trois mémoires et d'identifier l'élément (ensemble d'électrodes/clé) auquel il est connecté. Cet adressage sur 2 bits peut s'accommoder d'ailleurs d'une quatrième mémoire placée dans l'un ou l'autre des éléments du dispositif, pour accueillir des informations utiles aux traitements appliqués par le dispositif suivant l'invention.

Chacune des mémoires 12 et 22 de l'ensemble d'électrodes 1 et de la clé 3 respectivement contient aussi un code d'identification C1, C3 respectivement, propre à l'ensemble 1 et à la clé 3 respectivement. La mémoire 24 du module 2 contient les codes d'identification de la clé 3 et de l'ensemble d'électrodes 1 qui sont compatibles avec le module électronique 2, du point de vue du médicament contenu dans l'ensemble d'électrodes, de son programme d'administration enregistré dans le module et de la personne (médecin compétent ou patient) autorisée à mettre en oeuvre le traitement.

Ainsi le dispositif suivant l'invention assure-t-il, lorsqu'un certain médicament ne doit être administré que suivant un certain programme d'administration, sous l'autorité et la surveillance d'une certaine personne, que la triple condition de sécurité qui doit régir cette administration est bien remplie.

On se réfère maintenant à l'organigramme de la figure 4 pour décrire la procédure de vérification, suivant l'invention, de cette triple condition de sécurité.

Le programme de détection des connexions/déconnexions des éléments du dispositif suivant l'invention, décrit en liaison avec l'organigramme de la figure 3, étant exécuté en permanence par le microcontrôleur 23, en quelques microsecondes, celui-ci sait à tout instant s'il est connecté ou non. A la détection d'une connexion (pas $P_{12}$, figure 4), le microcontrôleur identifie l'élément connecté en lisant dans la mémoire de cet élément l'adresse $E_1E_2$ particulière à cet élément. Si l'élément connecté au module est reconnu par son adresse comme étant l'ensemble d'électrodes 1 (pas $P_{13}$), le programme recherche si la clé 3 a été préalablement connectée au module (dans le cas d'un traitement faisant intervenir cette clé) et reconnue par ce dernier comme compatible. Si ce n'est pas le cas, c'est que, lors de la préparation d'un traitement, le détenteur de la clé n'a pas présenté sa clé au module. Le microcontrôleur 23 commande alors l'affichage, sur l'écran 15 du module 2, du message "clé" pour rappeler au détenteur de cette clé qu'il doit d'abord la présenter au module 2. Le cycle décrit ci-dessus recommence jusqu'à ce que cette présentation soit faite (pas $P_{14}$). Le microcontrôleur contrôle ensuite (pas $P_{15}$) le code C3 contenu dans la mémoire 22 de la clé 3. Si celui-ci est reconnu et accepté par le module 2, l'écran d'affichage 15 de ce dernier présente le message "OK" pour informer le détenteur de la clé 3 qu'il est autorisé à mettre en oeuvre le dispositif suivant l'invention, au moins avec des ensembles d'électrodes chargés de certains médicaments.

Suivant un mode de réalisation particulier de l'invention, si le code C3 de la clé est acceptée par le module 2, on passe à un sous-programme de vérification du bon fonctionnement du dispositif, en phase d'administration d'un médicament, en particulier de sa capacité à commander l'intensité du courant "thérapeutique". Pour ce faire, la clé contient avantageusement une impédance Z simulant celle de la peau d'un patient.

Le module 2 débite alors dans cette impédance (pas $P_{16}$) un courant dont l'intensité est mesurée par le microcontrôleur 23. La mesure est comparée à des valeurs mises en mémoire. Si la comparaison révèle que le module fonctionne correctement, la séquence de vérification du code C1 de l'ensemble d'électrodes 1 peut commencer, dès que le module est retiré de la clé 3 et installé ensuite sur cet ensemble (pas $P_{17}$). Si, au contraire, la clé est rejetée par le module, le détenteur de cette clé en est averti par l'apparition du message "code" sur l'écran 15. Le cycle de vérification reprend à son début, jusqu'à ce que, le cas échéant, une clé soit acceptée par le module.

Si le code C1 de l'ensemble d'électrodes 1 monté sur le module n'est pas reconnu par ce dernier, le détenteur de cet ensemble en est averti par l'affichage du message "code" sur l'écran 15. Si, au contraire, ce code est reconnu par le microcontrôleur, le message "OK" apparaît sur cet écran pour indiquer que le traitement peut commencer.

Il apparaît maintenant que, grâce au dispositif suivant l'invention, la sécurité du patient est parfaitement assurée dans le cas où le traitement prévu exige que des précautions soient prises quant à la capacité de l'administrateur à mettre en oeuvre ce traitement, à l'identité du médicament administré et à l'adéquation du programme d'administration de ce médicament, tel qu'il doit être commandé par le module électronique. On remarquera en outre que le dispositif suivant l'invention comprend également des moyens de vérification du bon fonctionnement du module électronique, avant le lancement d'un traitement et du bon état de charge des piles, aussi bien avant que pendant ce traitement.

Bien entendu, l'invention n'est pas limitée au mode de réalisation décrit et représenté qui n'a été donné qu'à titre d'exemple. C'est ainsi que le programme d'administration de médicament peut être initialement enregistré ailleurs que dans le module électronique, c'est-à-dire soit dans la mémoire de la clé, soit dans le mémoire de l'ensemble d'électrodes. Quand le programme d'administration est initialement enregistré dans la clé, le traitement peut commencer, après déverrouillage du module par la clé, dès que le module est monté sur l'ensemble d'électrodes.

Le programme d'administration peut aussi être enregistré initialement dans la mémoire 12 de l'ensemble d'électrodes 1, qui embarque ainsi le programme associé au médicament qu'il contient. Le module lit le programme enregistré dans la mémoire 12 de l'ensemble 1 et l'exécute. Cette disposition permet de préprogrammer différemment les ensembles d'électrodes d'un jeu de tels ensembles devant être utilisés successivement, par exemple pour administrer au patient, de jour en jour, des doses croissantes ou décroissante du médicament.

Le programme d'administration peut encore être initialement enregistré dans la mémoire 22 de la clé 3. Dans ce cas, il doit être recopié dans la mémoire 24 du module 2 quand celui-ci est monté sur la clé, pendant la phase de reconnaissance de celle-ci. Le traitement est ainsi piloté uniquement par la personne qui détient la clé, médecin ou pharmacien par exemple. Après chargement de l'ensemble d'électrodes par cette personne, cet ensemble devient utilisable par le patient.

On a décrit ci-dessus le module électronique du dispositif suivant l'invention comme comprenant le code d'une seule clé et celui d'un seul ensemble d'électrodes, pour simplifier la description de son fonctionnement. Cependant, en variante, la mémoire du module pourrait contenir les codes de plusieurs clés pour autoriser plusieurs personnes également qualifiées à déclencher un traitement. Une solution alternative peut consister à attribuer le même code de clé à plusieurs personnes qualifiées, appartenant à un même centre de soins, par exemple.

De même, le module électronique 2 peut contenir en mémoire plusieurs codes d'ensemble d'électrodes, par exemple lorsqu'un même traitement implique l'utilisation d'un lot d'ensembles d'électrodes, mis en oeuvre successivement, les ensembles d'un même lot étant chargés de médicaments différents se complétant pour un traitement particulier.

Quand un traitement n'exige pas une surveillance par une personne de compétence particulière, du fait d'une absence de danger par exemple, la reconnaissance mutuelle des éléments du dispositif suivant l'invention ne fait plus intervenir alors que le code de l'ensemble d'électrodes, le module étant dûment programmé pour que le processus de reconnaissance de la clé soit supprimé.

De même, quand le traitement n'exige pas une surveillance de la compatibilité de l'ensemble d'électrodes avec le module électronique utilisé, du fait que le dispositif d'administration ne peut recevoir qu'un seul type d'électrodes par exemple, la reconnaissance mutuelle des éléments du dispositif ne fait plus intervenir que la clé et le module électronique.

Dans tout ce qui précède, on a décrit l'invention dans son application à l'administration transdermique de médicaments sous l'influence d'une force ionophorétique. Il est clair cependant que l'invention peut trouver également application à l'administration transdermique de médicaments par électroosmose.

## Revendications

**1.** Dispositif d'administration transdermique de médicament par ionophorèse ou électroosmose, comprenant au moins premièrement un ensemble (1) incluant une électrode (5) accolée à un réservoir (7) chargeable avec un médicament, et une contre-électrode (6), deuxièmement un module électronique (2) montable, de manière séparable, sur ledit ensemble (1) pour commander dans le temps l'intensité d'un courant électrique thérapeutique traversant, entre les deux électrodes (5, 6), le réservoir (7) et la peau d'un patient appliquée contre ledit réservoir, ledit module électronique (2) comprenant des moyens de commande (23) du fonctionnement du dispositif, caractérisé en ce qu'il comprend troisièmement une clé électronique (3) incluant une mémoire (22) chargée avec un code prédéterminé (C3) et des moyens de connexion temporaire (201, 202) au module électronique (2) pour la lecture de ce code (C3) par des moyens de lecture (28) présents dans ce module, les moyens de commande (23) dudit module étant sensibles au code (C3) de ladite clé pour autoriser sélectivement le fonctionnement du dispositif et en ce que la clé forme un élément distinct des deux autres.

2. Dispositif conforme à la revendication 1, caractérisé en ce que l'ensemble d'électrodes (1) comprend une mémoire électronique (12) chargée avec un code prédéterminé ($C_1$), en ce que lesdits moyens (28) comprennent des moyens de lecture dudit code ($C_1$) et en ce que lesdits moyens de commande (23) prennent aussi en compte le code ($C_1$) dudit ensemble (1) pour autoriser sélectivement le fonctionnement du dispositif.

3. Dispositif conforme à la revendication 1 ou 2, caractérisé en ce que l'ensemble d'électrodes (1) comprend un berceau (9) de réception du module (2) et des moyens de connexion électrique ($10_1$, $10_2$) à ce module.

4. Dispositif conforme à la revendication 1, 2 ou 3, caractérisé en ce que la clé (3) comprend un berceau (19) de réception du module (2) et des moyens de connexion électrique ($20_1$, $20_2$) à ce module.

5. Dispositif conforme à l'une quelconque des revendications 1 à 4, caractérisé en ce que l'ensemble d'électrodes (1) et la clé (3) comprennent chacun des moyens de couplage mécanique ($13_1$, $13_2$, ; $19_1$, $19_2$) coopérant avec des moyens mécaniques ($14_1$, $14_2$) du module pour assurer leur couplage temporaire à ce module.

6. Dispositif conforme à l'une quelconque des revendications 1 à 5, caractérisé en ce que l'ensemble d'électrodes (1) comprend une pile d'alimentation électrique (11) et des moyens pour assurer l'alimentation électrique du module (2) avec cette pile (11) lorsqu'ils sont connectés.

7. Dispositif conforme à l'une quelconque des revendications 1 à 6, caractérisé en ce que la clé (3) comprend une pile d'alimentation électrique (21) et des moyens pour assurer l'alimentation électrique du module (2) avec cette pile (21) lorsqu'ils sont connectés.

8. Dispositif conforme à l'une quelconque des revendications 1 à 7, caractérisé en ce que les moyens de commande sélective du fonctionnement du dispositif sont constitués par un microcontrôleur (23) et par une mémoire électronique (24) associée dans le module, ladite mémoire (24) étant chargée avec les codes (C1;C3) d'au moins un ensemble d'électrodes (1) et d'au moins une clé (3) faisant partie du dispositif, ledit microcontrôleur étant programmé pour lire le code contenu dans la mémoire (22) d'une clé (3) et dans celle (12) d'un ensemble d'électrodes (1) respectivement, pour les comparer aux codes correspondants enregistrés dans sa propre mémoire (24) et pour autoriser l'administration du médicament quand les codes (C1;C2) de ladite clé et dudit ensemble sont identiques aux codes correspondants enregistrés dans sa propre mémoire (24).

9. Dispositif conforme à la revendication 8, caractérisé en ce que le module (2) comprend des moyens (26, 27) de commande de l'alimentation électrique du microcontrôleur (23), ces moyens comprenant un régulateur (26) alimenté par la pile (11 ; 21) de la clé (3) ou de l'ensemble (1) quand l'un ou l'autre de ces éléments est couplé au module (2), et un circuit superviseur (27) alimenté par une pile d'alimentation électrique de sauvegarde (25) prévue pour alimenter le module quand il n'est pas alimenté par une pile (11 ; 21) de la clé (3) ou de l'ensemble (1), respectivement, le circuit superviseur (27) commandant sélectivement l'alimentation du microcontrôleur (23) soit par la sortie du régulateur (26) soit par la pile de sauvegarde (25).

10. Dispositif conforme à la revendication 9, caractérisé en ce que le microcontrôleur reçoit un signal numérique (LBO) du régulateur (26) et représentatif de ce que le régulateur est alimenté électriquement ou non, et un signal numérique ($\overline{PFO}$) venu du circuit superviseur (27) et représentatif de ce que sa tension d'alimentation ($V_s$) par le régulateur (26) est supérieure à un seuil prédéterminé, alors que la tension qui lui est délivrée par la pile (25) du module est aussi supérieure à un seuil prédéterminé, ou de ce que ces deux tensions sont inférieures aux seuils correspondants, le microcontrôleur étant programmé pour déduire des niveaux logiques des signaux (LBO, $\overline{PFO}$), la connexion ou la déconnexion du module (2) par rapport à l'un quelconque des éléments (1) et (3) du dispositif.

11. Dispositif conforme à la revendication 10, caractérisé en ce que le microcontrôleur (23) est aussi programmé pour tirer des niveaux logiques des signaux (LBO, $\overline{PFO}$) une information sur l'état de la pile (25) d'alimentation de sauvegarde du microcontrôleur (23) et sur l'état de la pile (11 ou 21) d'un élément (1 ou 3) éventuellement connecté au module (2).

12. Dispositif conforme à l'une quelconque des revendications 10 et 11, caractérisé en ce que le module électronique (2) comprend un écran d'affichage (15) de messages et d'icônes, cet écran étant commandé pour afficher des messages et des informations établis par le microcontrôleur (25) pendant l'exécution des programmes dont il est chargé.

13. Dispositif conforme à la revendication 12, caractérisé en ce que le microcontrôleur (25) est programmé, à la suite de la détection d'une connexion module (2)/clé (3) ou module (2)/ensemble d'électrodes (1), pour a) émettre un message d'alerte ("clé") sur l'écran (15) quand une connexion module (2)/ensemble (1) intervient sans connexion module (2)/clé (3) préalable, b) comparer le code (C3) en mémoire (22) de la clé (3) au code correspondant mis dans la mémoire (24) du module (2) lors d'une première connexion du module (2) à la clé (3), c) interdire tout traitement si les codes comparés ne sont pas identiques, d) comparer le code (C1) en mémoire (12) de l'ensemble d'électrodes (1) au code correspondant en mémoire (24) du module (2) lors d'une connexion ultérieure de l'ensemble et du module, si le traitement n'est pas interdit et e) déverrouiller le traitement si ces derniers codes sont identiques.

14. Dispositif conforme à la revendication 13, caractérisé en ce qu'il comprend un sous-programme de vérification du fonctionnement d'un programme de commande du courant thérapeutique passant entre les électrodes (5, 6) de l'ensemble (1) pendant l'exécution d'un traitement, l'exécution de ce sous-programme étant déclenché par la reconnaissance du code de la clé (3) par le module (2), une impédance électrique (Z) étant montée dans la clé (3) pour être alimentée par un courant électrique commandé par le module (2), pour simuler la peau pendant l'exécution dudit sous-programme.

15. Dispositif conforme à l'une quelconque des revendications 1 à 14, comprenant des moyens (12 ; 22; 24) pour mémoriser un programme de commande de l'évolution dans le temps de l'intensité du courant thérapeutique, caractérisé en ce que lesdits moyens de mémoire sont localisés dans l'un quelconque des trois éléments (1, 2, 3) du dispositif.

16. Dispositif conforme à la revendication 15, dans lequel lesdits moyens de mémoire (22) sont disposés dans la clé (3), caractérisé en ce qu'il comprend des moyens (23) pour commander le transfert du programme contenu dans la mémoire (22) de la clé (3), dans la mémoire (24) du module électronique (2), lors de la connexion clé (3)/module (2).

## Patentansprüche

1. Vorrichtung zur transdermalen Verabreichung eines Arzneimittels durch Iontophorese oder Elektroosmose, umfassend mindestens erstens eine Einheit (1), die eine Elektrode (5), die an einen mit einem Arzneimittel ladbaren Behälter (7) angefügt ist, und eine Gegenelektrode (6) enthält, und zweitens ein auf dieser Einheit (1) trennbar montierbares elektronisches Modul (2) zur zeitlichen Steuerung der Stärke eines elektrischen Therapiestroms, der zwischen den beiden Elektroden (5, 6) den Behälter (7) und die an den Behälter angelegte Haut eines Patienten durchquert, wobei dieses elektronische Modul (2) Mittel (23) zur Steuerung des Betriebs der Vorrichtung aufweist, dadurch gekennzeichnet, daß sie drittens einen elektronischen Schlüssel (3) umfaßt, der einen mit einem zuvor festgelegten Code ($C_3$) geladenen Speicher (22) und Mittel ($20_1$, $20_2$) zur zeitweiligen Verbindung mit dem elektronischen Modul (2) für das Lesen dieses Codes ($C_3$) durch in diesem Modul vorhandene Lesemittel (28) enthält, wobei die Steuermittel (23) des Moduls gegenüber dem Code ($C_3$) des Schlüssels empfindlich sind, um den Betrieb der Vorrichtung selektiv zuzulassen, und dadurch, daß der Schlüssel ein von den anderen Elementen getrenntes Element bildet.

2. Vorrichtung nach Anspruch 1, dadurch gekennzeichnet, daß die Elektrodeneinheit (1) einen elektronischen Speicher (12) aufweist, der mit einem vorbestimmten Code ($C_1$) geladen ist, daß die Mittel (28) Mittel zum Lesen dieses Codes ($C_1$) aufweisen und daß die Steuermittel (23) auch den Code ($C_1$) dieser Einheit (1) berücksichtigen, um den Betrieb der Vorrichtung selektiv zuzulassen.

3. Vorrichtung nach Anspruch 1 oder 2, dadurch gekennzeichnet, daß die Elektrodeneinheit (1) eine Halterung (9) zur Aufnahme des Moduls (2) und Mittel ($10_1$, $10_2$) zum elektrischen Anschluß an das Modul aufweist.

4. Vorrichtung nach Anspruch 1, 2 oder 3, dadurch gekennzeichnet, daß der Schlüssel (3) eine Halterung (19) zur Aufnahme des Moduls (2) und Mittel ($20_1$, $20_2$) zum elektrischen Anschluß an dieses Modul aufweist.

5. Vorrichtung nach einem der Ansprüche 1 bis 4, dadurch gekennzeichnet, daß die Elektrodeneinheit (1) und der Schlüssel (3) jeweils mechanische Kopplungsmittel ($13_1$, $13_2$; $19_1$, $19_2$) aufweisen, die mit mechanischen Mitteln ($14_1$, $14_2$) des Moduls zu-

sammenwirken, um ihre zeitweilige Kopplung mit diesem Modul zu gewährleisten.

6. Vorrichtung nach einem der Ansprüche 1 bis 5, dadurch gekennzeichnet, daß die Elektrodeneinheit (1) eine elektrische Versorgungsbatterie (11) und Mittel besitzt, um die elektrische Versorgung des Moduls (2) mit dieser Batterie (11) zu gewährleisten, wenn sie verbunden sind.

7. Vorrichtung nach einem der Ansprüche 1 bis 6, dadurch gekennzeichnet, daß der Schlüssel (3) eine elektrische Versorgungsbatterie (21) und Mittel besitzt, um die elektrische Versorgung des Moduls (2) mit dieser Batterie (21) zu gewährleisten, wenn sie verbunden sind.

8. Vorrichtung nach einem der Ansprüche 1 bis 7, dadurch gekennzeichnet, daß die Mittel zur selektiven Steuerung des Betriebs der Vorrichtung aus einem Mikrosteuergerät (23) und aus einem in dem Modul zugeordneten elektronischen Speicher (24) bestehen, wobei dieser Speicher (24) mit den Codes (C1; C3) mindestens einer Elektrodeneinheit (1) und mindestens eines Schlüssels (3) geladen ist, der Teil der Vorrichtung bildet, wobei das Mikrosteuergerät programmiert ist, um den im Speicher (22) eines Schlüssels (3) bzw. im Speicher (12) einer Elektrodeneinheit (1) enthaltenen Code zu lesen, um sie mit den entsprechenden, in seinem eigenen Speicher (24) gespeicherten Codes zu vergleichen und um die verabreichung des Arzneimittels zuzulassen, wenn die Codes (C1; C2) des Schlüssels und der Einheit mit den entsprechenden, in seinem eigenen Speicher (24) gespeicherten Codes identisch sind.

9. Vorrichtung nach Anspruch 8, dadurch gekennzeichnet, daß das Modul (2) Mittel (26, 27) zur Steuerung der elektrischen Versorgung des Mikrosteuergeräts (23) aufweist, die einen Regler (26), der durch die Batterie (11; 21) des Schlüssels (3) oder der Einheit (1) versorgt wird, wenn das eine oder das andere dieser Elemente mit dem Modul (2) gekoppelt ist, und einen Überwachungskreis (27) aufweisen, der durch eine Batterie (25) zur elektrischen Sicherungsversorgung versorgt wird, die vorgesehen ist, um das Modul zu versorgen, wenn es nicht durch die Batterie (11; 21) des Schlüssels (3) bzw. der Einheit (1) versorgt wird, wobei der Überwachungskreis (27) die Versorgung des Mikrosteuergeräts (23) selektiv entweder über den Ausgang des Reglers (26) oder über die Sicherungsbatterie (25) steuert.

10. Vorrichtung nach Anspruch 9, dadurch gekennzeichnet, daß das Mikrosteuergerät vom Regler (26) ein digitales Signal (LBO) erhält, das dafür repräsentativ ist, daß der Regler elektrisch versorgt wird oder nicht, sowie ein vom Überwachungskreis (27) kommendes digitales Signal ($\overline{\text{PFO}}$), das dafür repräsentativ ist, daß die Spannung (Vs) seiner Versorgung durch den Regler (26) über einer vorbestimmten Schwelle liegt, während die ihm von der Batterie (25) des Moduls gelieferte Spannung ebenfalls über einer vorbestimmten Schwelle liegt, oder dafür, daß diese beiden Spannungen unter den entsprechenden Schwellen liegen, wobei das Mikrosteuergerät programmiert ist, um von den logischen Pegeln der Signale (LBO, $\overline{\text{PFO}}$) das Verbundensein oder Getrenntsein des Moduls (2) gegenüber einem beliebigen der Elemente (1) und (3) der Vorrichtung abzuleiten.

11. Vorrichtung nach Anspruch 10, dadurch gekennzeichnet, daß das Mikrosteuergerät (23) auch programiert ist, um aus den logischen Pegeln der Signale (LBO, $\overline{\text{PFO}}$) eine Information über den Zustand der Batterie (25) zur Sicherungsstromversorgung des Mikrosteuergeräts (23) und über den Zustand der Batterie (11 oder 21) eines ggf. an das Modul (2) angeschlossenen Elements (1 oder 2) zu ziehen.

12. Vorrichtung nach einem der Anssprüche 10 und 11, dadurch gekennzeichnet, daß das elektronische Modul (2) ein Display (15) zur Anzeige von Mitteilungen und Bildzeichen besitzt, das so gesteuert wird, um Mitteilungen und Informationen anzuzeigen, die von dem Mikrosteuergerät (25) während der Durchführungs der Programme, mit denen es geladen ist, erstellt werden.

13. Vorrichtung nach Anspruch 12, dadurch gekennzeichnet, daß das Mikrosteuergerät (25) programmiert ist, um auf die Erfassung einer Verbindung Modul (2) / Schlüssel (3) oder Modul (2) / Elektrodeneinheit (1) hin a) auf dem Display (15) eine Warnmitteilung ("Schlüssel") zu senden, wenn eine Verbindung Modul (2) / Einheit (1) ohne vorhergehende Verbindung Modul (2) / Schlüssel (3) stattfindet, b) den Code (C3) im Speicher (22) des Schlüssels (3) mit dem entsprechenden Code zu vergleichen, der bei einer ersten Verbindung des Moduls (2) mit dem Schlüssel (3) im Speicher (24) des Moduls (2) gespeichert wurde, c) jede Behandlung zu sperren, wenn die verglichenen Codes nicht identisch sind, d) den Code (C1) im Speicher (12) der Elektrodeneinheit (1) mit dem entsprechenden Code im Speicher (24) des Moduls (2) bei einer späteren Verbindung der Einheit und des Moduls zu vergleichen, wenn die Behandlung nicht gesperrt ist, und e) die Behandlung freizugeben, wenn diese Codes identisch sind.

14. Vorrichtung nach Anspruch 13, dadurch gekennzeichnet, daß sie ein Unterprogramm zur Prüfung der Arbeitsweise eines Programms zur Steuerung des während der Durchführung einer Behandlung zwischen den Elektroden (5, 6) der Einheit (1) fließenden Therapiestroms aufweist, wobei die Durchführung dieses Unterprogramms dadurch ausgelöst wird, daß der Code des Schlüssels (3) vom Modul (2) erkannt wird, wobei im Schlüssel (3) eine elektrische Impedanz (Z) geschaltet ist, um zur Simulation der Haut während der Durchführung des Unterprogramms durch einen vom Modul (2) gesteuerten elektrischen Strom versorgt zu werden.

15. Vorrichtung nach einem der Ansprüche 1 bis 14 mit Mitteln (12; 22; 24) zum Speichern eines Programms zur Steuerung der zeitlichen Änderung der Stärke des Therapiestroms, dadurch gekennzeichnet, daß diese Speichermittel in einem beliebigen der drei Elemente (1, 2, 3) der Vorrichtung angeordnet sind.

16. Vorrichtung nach Anspruch 15, in der diese Speichermittel im Schlüssel (3) angeordnet sind, dadurch gekennzeichnet, daß sie Mittel (23) zur Auslösung der Übertragung des im Speicher (22) des Schlüssels (3) enthaltenen Programms in den Speicher (24) des elektronischen Moduls (2) bei der Verbindung Schlüssel (3) / Modul (2) aufweist.

## Claims

1. Device for transdermal administration of medication by ionophoresis or electro-osmosis comprising at least firstly a set of electrodes (1) including an electrode (5) joined to a reservoir (7) that can be charged with a medication and a counter-electrode (6), secondly an electronic module (2) separably mounted on said set of electrodes (1) to control a therapeutic electric current flowing, between the two electrodes (5, 6), through the reservoir (7) and the skin of a patient applied against said reservoir, said electronic module (2) comprising means (22) for controlling the operation of the device, characterised in that it further comprises thirdly an electronic key (3) including a memory (22) loaded with a predetermined code (C3) and means ($20_1$, $20_2$) for temporary connection to the electronic module (2) to read this code (C3) by reading means (28) present in the module, the control means (23) of said module being responsive to the code (C3) of said key to authorise selectively the operation of the device and in that the key forms an element which is separate from the two others.

2. Device according to claim 1 characterised in that the set of electrodes (1) includes an electronic memory (12) loaded with a predetermined code ($C_1$), in that said means (28) include means for reading said code (C1) and in that said control means (23) also allow for the code (C1) of said system (1) when selectively authorising the operation of the device.

3. Device according to claim 1 or claim 2 characterised in that the set of electrodes (1) includes a cradle (9) receiving the module (2) and means ($10_1$, $10_2$) for making electrical connections to this module.

4. Device according to claim 1 or claim 2 or claim 3 characterised in that the key (3) includes a cradle (19) to receive the module (2) and means ($20_1$, $20_2$) for establishing electrical connections to this module.

5. Device according to any one of claims 1 to 4 characterised in that the set of electrodes (1) and the key (3) each include mechanical connection means ($13_1$, $13_2$; $19_1$, $19_2$) cooperating with mechanical means ($14_1$, $14_2$) of the module to connect them temporarily to this module.

6. Device according to any one of claims 1 to 5 characterised in that the set of electrodes (1) includes an electrical power supply battery (11) and means for supplying electrical energy to the module (2) from the battery (11) when they are connected.

7. Device according to any one of claims 1 to 6 characterised in that the key (3) includes an electrical power supply battery (21) and means for supplying electrical power to the module (2) from the battery (21) when they are connected.

8. Device according to any one of claims 1 to 7 characterised in that the means for selectively controlling operation of the device comprise a microcontroller (23) and an associated electronic memory (24) in the module, said memory (24) being loaded with the codes (C1; C3) of at least one set of electrodes (1) and at least one key (3) forming part of the device, said micrcontroller being programmed to read the code contained in the memory (22) of a key (3) and in that (12) of a set of electrodes (1), respectively, to compare them with the corresponding codes stored in its own memory (24) and to authorise administration of the medication when the codes (C1; C3) of said key and of said set of electrodes are identical to the corresponding codes stored in its own memory (24).

9. Device according to claim 8 characterised in that the module (2) includes means (26, 27) for controlling the supply of electrical energy to the microcontroller (23), these means including a regulator (26) supplied with energy by the battery (11; 21) of the key (3) or of the set of electrodes (1) when one or other of these components is connected to the module (2), and a supervisor circuit (27) supplied with energy by a back-up electrical power supply battery (25) adapted to supply energy to the module when it is not supplied with energy by a battery (11; 21) of the key (3) or of the set of electrodes (1), respectively, the supervisor circuit (27) selectively controlling the supply of energy to the microcontroller (23) either from the output of the regulator (26) or from the back-up battery (25).

10. Device according to claim 9 characterised in that the microcontroller receives a digital signal (LBO) from the regulator (26) indicating whether the regulator is supplied with electrical energy or not and a digital signal ( $\overline{PFO}$ ) from the supervisor circuit (27) and indicating whether the power supply voltage (Vs) which is supplied by the regulator (26) is above a predetermined threshold while the voltage supplied to it by the battery (25) of the module is also above a predetermined threshold or that these two voltages are below the corresponding thresholds, the microcontroller being programmed to deduce from the logical levels of the signals (LBO, $\overline{PFO}$) whether the module (2) is connected to or disconnected from either of the components (1) and (3) of the device.

11. Device according to claim 10 characterised in that the microcontroller (23) is also programmed to derive from the logic levels of the signals (LBO, $\overline{PFO}$) information as to the condition of the back-up power supply battery (25) of the microcontroller (23) and the condition of the battery (11 or 21) of a component (1 or 3) if connected to the module (2).

12. Device according to claim 10 or claim 11 characterised in that the electronic module (2) includes a screen (15) for displaying messages and icons, this screen being controlled to display messages and information established by the microcontroller (23) during execution of the programmes for which it is responsible.

13. Device according to claim 12 characterised in that the microcontroller (23) is programmed, following detection of a module (2)/key (3) or module (2)/electrode (1) connection, to a) send an alert message ("key") to the screen (15) when a module (2)/electrode (1) connection is present with no prior module (2)/key (3) connection, b) compare the code (C3) in the memory (22) of the key (3) to the corresponding code placed in the memory (24) of the module (2) upon first connection of the module (2) to the key (3), c) prohibit treatment if the codes compared are not identical, d) compare the code (C1) in the memory (12) of the set of electrodes (1) to the corresponding code in the memory (24) of the module (2) upon subsequent connection of the set of electrodes and the module, if the treatment is not prohibited, and e) enabling the treatment if the latter codes are identical.

14. Device according to claim 13 characterised in that it includes a subroutine for verifying the operation of a programme for controlling the therapeutic current flowing between the electrodes (5, 6) of the set of electrodes (1) during execution of a treatment, execution of this subroutine being initiated by recognition of the code of the key (3) of the module (2), an electrical impedance (Z) being mounted in the key (3) to be energised by an electrical current commanded by the module (2) to simulate the skin during the execution of said subroutine.

15. Device according to any one of claims 1 to 14 including means (12; 22; 24) for storing in memory a programme for controlling the therapeutic current, characterised in that said means for storage in memory are located in any one of the three components (1, 2, 3) of the device.

16. Device according to claim 15 wherein said means for storage in memory (22) are in the key (3), characterised in that it comprises means (23) for controlling the transfer of the programme contained in the memory (22) of the key (3) into the memory (24) of the electronic module (2) upon key (3)/module (2) connection.

FIG.:1

FIG.:2

DEBUT

$P_1$ — ETAT INITIAL:
LBO=0
$\overline{PFO}$=0

ETAT INITIAL:
LBO=1
PFO=1

$P_2$ — CONNEXION

$P_6$ — DECONNEXION

$P_{10}$ — CHUTE DE Vbat

LBO

$\overline{PFO}$ LBO

LBO

$P_3$ — LBO=1

$P_7$ — PFO=0
LBO=0

PFO=1
LBO=0

$P_4$ — $\overline{PFO}$=1

$\overline{PFO}$=0

$P_8$ — SURVEILLAN-
CE PILE 25

AFFICHAGE
ICONE "PILE"

$P_5$ — MODULE CONNECTE

SURVEILLAN-
CE PILE 25

$P_9$ — CONFORME
?

OUI

NON

$P_{11}$

MODULE
DECONNECTE

FIG.: 3

FIN

FIG.:4